# EUROPEAN PATENT APPLICATION

(11) **EP 4 417 109 A1**
(43) Date of publication of application: **21.08.2024**
(21) Application number: 23157295.9
(22) Date of filing: 17.02.2023
(51) Int. Cl.: A61B 5/00

(54) **METHOD AND DEVICE FOR USER STATE DATA ACQUISITION**

(71) Applicant: Givaudan SA, 1214 Vernier (CH)
(72) Inventor: The designation of the inventor has not yet been filed
(74) Representative: Global Patents

(57) **Abstract**

A computer-implemented method of acquiring user state data and underlying signal data in a wearable device, the wearable device comprising a main processor unit and a lower-power processor unit, the method comprising: accessing an indicator of device sub-state; if the device sub-state is an unknown device sub-state, proceeding with the main processor unit switched on, and acquiring and processing in the main processor unit a first plurality of sensor signals to acquire user state data; and if the device sub-state is a known device sub-state, proceeding with the main processor unit switched off, and acquiring and processing in the lower-power processor unit a second sensor signals which are used for the known device sub-state to acquire user state data.

## Description

### Field of the Invention

The present invention relates to acquisition of user state data and underlying signal data. In particular, the present invention relates to a computer-implemented method of acquiring user state data in a wearable device and to a wearable device for acquisition of user state data.

### Background of the Invention

Modern wearable technology is able to discretely capture data concerning a user's mood, movement, and well-being. Such technology enables users to track and understand their own behaviour and habits. A wearable device that tracks physical activity may help a user understand how much exercise they are performing on a daily basis and to identify patterns in their activity levels. This is often useful for setting and achieving fitness goals, as well as for identifying any potential health concerns that may need to be addressed.

Wearable technology may be used to gather data for research purposes. Studies may use wearable devices to track the movement and well-being of a group of participants over a period of time in order to better understand the relationship between physical activity and mental health. This type of research can lead to a deeper understanding of how certain behaviours and habits affect our overall well-being, which can in turn inform the development of new interventions and treatments.

Wearable technology may be used to support personalized health care. A wearable device that tracks a user's sleep patterns and heart rate could be used to alert a healthcare provider to any potential issues or changes in a patient's health. This type of real-time monitoring may allow healthcare providers to intervene earlier and potentially prevent more serious problems from developing.

The emotional state or mood of a user may be inferred from, e.g., biometric user data and may be highly influenced by other seemingly unrelated bodily processes and activities. However, it is only through the collection of a comprehensive set of diverse but coherent signals over long continuous periods of monitoring a user that reliable correlates may be identified that may be used to measure user data objectively.

In order to find reliable correlates, the data that are collected must remain unbiased by conscious influence. That is, the means of collection must be sufficiently small, light, and unobtrusive that the subject rapidly becomes unaware of its presence - effectively a "fit and forget" approach. Success in collection of such datasets presents potential ethical issues, related to the inadvertent collection of information which might be considered private and/or embarrassing by the subject of the measurements.

The above factors, taken together, present a significant technical challenge which cannot be met by existing technology. Today, clinical-grade biometric and kinesiological data may be collected over extended periods of time, but only using relatively large, fixed equipment (e.g., located in hospitals, laboratories, clinics, or gyms). Conversely, wearable devices or apparatuses (e.g., watches, bands, patches) may collect a variety of non-clinical, but continuous biometric and kinesiological data outside of a fixed location. However, such wearable devices are hampered with unavoidably rapid depletion of available on-board power supplies and data storage capabilities. In neither type of scenario may the means of measurement be considered sufficiently unobtrusive as to become forgettable. Therefore, existing techniques offer no consideration to providing additional measures to preserve privacy.

The inventors have therefore come to the realisation that there is need for techniques and equipment that enable unintrusive acquisition of user state (e.g., biometric and kinesiological) data.

### Summary of the Invention

The invention is defined in the independent claims, to which reference should now be made. Further features are set out in the dependent claims.

According to an aspect of the invention, there is provided a computer-implemented method of acquiring user state data in a wearable device. The wearable device or apparatus comprises a main (higher power) processor unit and a lower-power processor unit. Also referred to as controllers or nodes, the processor units may either be on separate chips or part of a multi core chip. Regardless of their configuration, the processor units may be separated into a main processor unit, for higher-power processing, and a lower-power processor unit, for lower-power processing.

The method of acquiring user state data comprises accessing an indicator of device sub-state. Optionally, the lower-power processor unit accesses the indicator. Optionally, the main processor unit produces the indicator (when the main processor unit is able to successfully classify the device sub-state). The device sub-state includes a predefined mode of operation that sets the wearable device's operational parameters. The device sub-state comprises, for instance, an indication of which sensors are active, memory capabilities of the device, and battery capabilities of the device.

If the device sub-state is an unknown (and/or undefined) device sub-state, the method comprises proceeding with the main processor unit switched on. The method then comprises acquiring and processing - in the main processor unit - a first plurality of sensor signals to acquire user state data (that is, data indicating the state of the user). The first plurality of sensor signals is acquired from a first plurality of sensors. The first plurality of sensor signals is used for any unknown device sub-state. The first plurality of sensors may correspond to all sensors available to the wearable device, or any subgroup thereof.

If the device sub-state is a known (and/or defined) device sub-state, the method comprises proceeding with the main, higher-power processor unit switched off. The method then comprises acquiring and processing - in the lower-power processor unit - second sensor signals, which are used for the known device sub-state to acquire user state data. That is, the known device sub-state indicates the second sensor signals that are to be acquired, where the second sensor signals form, in part, the user state data. The second sensor signals are acquired from a second plurality of sensors (or, alternatively, a single sensor). In some cases, the second sensor signals may be in the form of a second plurality of sensor signals. In other cases (for instance, during deep sleep of the user), the second sensor signals may be a single sensor signal.

Optionally, the second sensor signals comprise fewer sensor signals than the first plurality of sensor signals. Alternatively, if it is an inherently "noisy" state, the second sensor signals may comprise the same number of sensor signals as the first plurality of sensor signals.

Of course, where a single sensor may be configured to output multiple sensor signals (e.g., a three-axis accelerometer may be configured to output three signals, one for each orthogonal axis), the absolute number of sensors used for the first plurality of sensor signals may be the same as that used for the second sensor signals, and only the number of sensor signals may vary (e.g., the three-axis accelerometer may be configured to output only a z-component of acceleration). Naturally, sensors from the second plurality of sensors may also be present in the first plurality of sensors (and vice versa).

In separating functionality to the main processor unit and the lower-power processor unit on the basis of device sub-state, the wearable device implementing the method ensures that no needless acquisition of data occurs while still providing continuous monitoring of the user. That is, in the event that a device sub-state comprises a device profile associated with the user being in a particular state where it is desirable to acquire particular biometric and kinesiological data, the method may instruct activation of only the required sensors (the second plurality of sensors). Conversely, in the event that the device sub-state is unknown, it is desirable to capture more data, for example all data that may be relevant (even if - at a later date - it is established that only a subset of this data is relevant to the eventual, classified device sub-state). Accordingly, the method may involve activation of all available sensors (the first plurality of sensors).

In proceeding with the main processor unit switched on only when required (when the device sub-state is unknown), unnecessary consumption of battery resources is avoided. That is, the main, higher-power processor unit may execute power-intensive processing to, e.g., perform classification on acquired data, and may facilitate the power-intensive data capture from more or all available sensors. Where such power-intensive processing and data capture is not necessary, functionality may pass to the lower-power processor unit, which does not need to execute the same power-intensive processing, and which may control data capture only from a relevant subset of sensors (the second plurality of sensors).

By reducing the unnecessary consumption of battery resources, the method may allow for significant reduction in the form-factor of wearable devices, in turn allowing for a "fit and forget" approach to data acquisition. This is particularly advantageous for continuous capture of clinical-grade biometric and kinesiological data, which is conventionally only obtainable using unwieldy hardware.

Moreover, by associating a known device sub-state to particular subset of sensor signals, the method provides measures to preserve privacy of the user. For instance, where the device sub-state indicates that location data of the user is not to be collected (e.g., in the event that the user is predicted to be in the vicinity of their home or workplace), the method may suspend data collection. Similarly, the skilled reader may envisage private user activities (as indicated by the device sub-state) for which suspension of data collection is desirable.

Optionally, the method may cause the main processor unit to process the first plurality of sensor signals by using at least one pretrained machine learning (ML) model to output a classification of the user state and/or a resultant state of the user. In this way, the method may instruct the higher-powered main processor unit to execute ML models, these models being capable of classification of previously unknown states. The resultant state of the user may be summarised as the collective classification of the user properties, taking into account, for instance, the activity of the user, the physiological properties of the user, environmental properties, and device properties.

Optionally, the method may cause the main processor unit to process the first plurality of sensor signals by using an initial pretrained ML model followed by a subsequent pretrained ML model. In this way, the main processor unit may pass the first plurality of sensor signals through the initial trained machine learning model to output a coarse classification of the user state according to pre-determined sub-states. The main processor unit may then pass the coarse classification of the user state and at least a portion of the first plurality of sensor signals (and/or data derived therefrom) through the subsequent trained machine learning model to output a resultant state of the user. It may be that the coarse classification is sufficiently representative of the state at hand, and therefore the subsequent classification is unnecessary - in this way, the wearable need not consume unnecessary resources in performing multiple classifications. The subsequent classification may be unnecessary, for instance if a confidence threshold of classification is above a predetermined value.

This process may involve storing the first plurality of sensor signals in a buffer, for access using the initial trained model and or the subsequent trained model. Following coarse classification, the buffer may be cleared to avoid unnecessary storage consumption.

Optionally, the method may cause the main processor unit to determine the confidence of the resultant state of the user. When the confidence of this classification is below a predetermined threshold, the main processor unit may pass the first plurality of sensor signals to a memory of the wearable device for further investigation (at a later time). The main processor unit may then continue to acquire the first plurality of sensor signals. That is, in the event that classification of the user's state is not possible, the method may cause the wearable device to store the data from the first plurality of sensors, which may be used for later investigation. Later investigation may be performed on-board when the device is equipped with extra data that may increase classification confidence, or may be performed by an external processing device.

Optionally, the method may cause the main processor unit to update adaptive parameters of any trained machine learning models following output of the classification of the user state and or/the resultant state of the user. In this way, the method allows for improved accuracy of user state classification during the next iteration of classification.

Optionally, the method may cause the lower-power processor unit to detect whether there is an anomaly when processing the second sensor signals (from the second plurality of sensors). An anomaly in this context is any divergence from expected values for the current state of the device, thereby potentially indicating a change of state. Following detection of an anomaly, the method may cause the lower-power processor unit to initialise the main processor unit. That is, the main processor unit is switched on (from an off or a standby state). The method may then cause the lower-power processor unit to pass the second sensor signals for processing in the main processor unit (e.g., passing the signals to a buffer for access by the main processor unit). The main processor unit may then process the second sensor signals. In this way, the power-intensive processing capabilities of the main processor unit may be used to classify otherwise unclassified data. Optionally, the main processor unit may supplement the second sensor signals with additional data (some or all of the first plurality of sensor data) acquired from the first plurality of sensors.

Optionally, the method may cause the lower-power processor unit to detect an anomaly by, first, passing at least a portion of the second sensor signals (and/or data derived therefrom) through an anomaly detection algorithm. This enables the lower-power processor unit to detect divergence from predetermined or prestored expected values for the current (known) device sub-state. The lower-power processor unit then detects the anomaly when a confidence level of the anomaly detection algorithm exceeds a predefined threshold. That is, the lower-power processor unit detects the anomaly when the confidence level of the anomaly detection algorithm is higher than a threshold, meaning that the anomaly detection algorithm is confident that it has detected an anomaly compared to what the algorithm was expecting for that given state. Anomaly detection operating parameters and thresholds may be different for each state as, in each state, a certain subset of parameters is considered to be more important than others.

Optionally, the method may cause the lower-power processor unit to update adaptive parameters of the anomaly detection algorithm following detection of an anomaly. In this way, the method allows for improved accuracy of anomaly detection during subsequent iterations of data acquisition.

Optionally, the method may, following acquisition of user state data, cause the wearable device to discard at least a portion of the user state data. The discarded portion may be the portion of the user state data deemed to be irrelevant for the known device sub-state. The relevance of the user state data to the known device sub-state may be preconfigured for each device sub-state. As an example, it may be desirable to know that a user is within a particular state (e.g., sleeping), which may be indicated via lack of movement from sensor signals; it is therefore necessary to collect movement data, but is not necessary (for classification purposes) to retain the same data.

Optionally, the method may cause the main processor unit to switch on when the indicator of the device sub-state is accessed. Then, when the device sub-state is unknown, the main processor unit may be left on. Similarly, when the device sub-state is known, the main processor unit may be switched off. In this way, the method ensures that the main processor unit is always switched on at the beginning of each user state data acquisition cycle.

Optionally, the method may cause the wearable device to acquire the sensor signals (that is, the first plurality of sensor signals and/or the second sensor signals) - at least in part - from any or all of the following: biometric sensors, for sensing biometric signals of the user (such as an ECG sensor, EEG, EMG, EOG, fNIRS, photoplethysmography sensor, electrodermal activity sensor, temperature, chemical composition, bioimpedance, skin moisture content, spectroscopic sensor, imaging sensor); environmental sensors, for sensing local environmental properties (such as an ambient temperature, pressure, acoustic, gas composition and optical properties, humidity, EMF emissions); communication (such as a Bluetooth receiver, WiFi, LORA, Zigbee, NFC); and kinematic sensors (such as an accelerometer, magnetometer, gyroscope, motion, and/or tilt-switch).

Optionally, the user state data acquired by the wearable device may be a resultant state of the user. The resultant state of the user may be determined according to any or all of the following sub-states: activity sub-state of the user; biometric (or physiological) sub-state of the user; environmental sub-state; and the device sub-state.

Optionally, the resultant state of the user may comprise an emotional profile of the user. The emotional profile may be a classification of the current emotional state of the user (that is, the emotional state of the user at the time of classification). The emotional profile may be included within the resultant state of the user as a tabular collection of marked and scored emotional descriptors or in any other suitable format. As an example, the emotional profile may be provided as scores for predetermined emotional descriptors (such as "excited", "angry", "stressed", "sad", "bored", tired", "calm", "happy", etc.). Optionally, the emotional profile of the user may be provided as an output from at least one pretrained ML model (such as the pretrained ML model(s) used for classification of the user state and/or a resultant state of the user). The use of an emotional profile enables contemporaneous classification of the user's mood during activities.

Optionally, the method may cause the wearable device to transmit user state data (and/or any data derived therefrom) to an external processing device. The wearable device may transmit the data using a communication subsystem. The external processing device may then further process the data as required. This is advantageous in that the wearable device may not be able to accurately classify the user state, and the increased processing power offered by an exemplary external processing device (for instance, a PC) may offer more intensive classification capabilities. Moreover, in the event that the wearable device may not be able to classify the user state as it has not encountered this state before (and thus classification will inevitably be inaccurate), transmission to an external processing device allows for model generation in view of this new user state (e.g., training of a new ML model); the new model may then be installed on the wearable for subsequent classifications of this state. The specific aspects of the data to be transmitted may be determined based on the end use of the wearable device and may include high level parameters and resultant states, such as user states and classifications, or lower-level data, such as raw data form the sensors (which is especially advantageous in cases where no classification is possible on-board).

Optionally, the method may cause the wearable device to acquire signals (that is, the first plurality of sensor signals and/or the second sensor signals) - at least in part - from an external device. In this way, classification is not limited in respect of on-board sensors. Rather, the functionality of the wearable device may be expanded in view of additional sensors. For instance, where the user is in the gym, Bluetooth-enabled gym equipment (beacons) may support classification of gym-related user-states (such as swimming indoors).

Embodiments of another aspect of the invention include a wearable device having a main processor unit, a lower-power processor unit, and a memory. The main processor unit and the lower-power processor unit may be configured to execute the steps of a method of acquiring user state data as variously explained above.

Embodiments of another aspect of the invention include a system comprising a wearable device and an external device. The wearable device comprises a main processor unit, a lower-power processor unit, and a memory; and the main processor unit and the lower-power processor unit may be configured to execute the steps of a method of acquiring user state data as variously explained above. The external device comprises transmission means configured to transmit sensor signals to the wearable device. The external device may also comprise reception means, configured to receive transmissions form the wearable device. For instance, the reception means may be configured to receive setting values and/or starting signals.

Embodiments of another aspect of the invention include a computer program which, when executed by a wearable device, causes the wearable device to be configured to execute the steps of a method of acquiring user state data.

Embodiments of another aspect of the inventive include a computer-readable medium storing a computer program, which when executed by a wearable device, causes the wearable device to be configured to execute the steps of a method of acquiring user state data. The computer-readable medium may be non-transitory.

The invention may be implemented in digital electronic circuitry, or in computer hardware, firmware, software, or in combinations thereof. The invention may be implemented as a computer program or a computer program product, i.e., a computer program tangibly embodied in a non-transitory information carrier, e.g., in a machine-readable storage device or in a propagated signal, for execution by, or to control the operation of, one or more hardware modules. A computer program may be in the form of a stand-alone program, a computer program portion, or more than one computer program, and may be written in any form of programming language, including compiled or interpreted languages, and it may be deployed in any form, including as a stand-alone program or as a module, component, subroutine, or other unit suitable for use in a data processing environment.

The firmware and/or software of the wearable device may be programmed using a variety of standard methods such as wired connection, wireless and over the air connection. The specific trial setup and/or usage parameters may also be programmed via a secondary device (such as a PC, Tablet, via Web interface, etc.) connected to the wearable device via these methods.

The invention is described in terms of particular embodiments. Other embodiments are within the scope of the following claims. For example, the steps of the invention may be performed in a different order and still achieve desirable results.

In summary, a method implemented in a wearable device comprising an integrated array of sensors (e.g., biometric, kinesiological, and environmental) may perform classification (e.g., using embedded artificial intelligence (Al)) in a manner than efficiently manages power consumption and data storage (and, in turn, improving privacy measures). This enables a reliable detection of - and changes in - the emotional state, mood, health, and well-being of the wearer.

### Brief Description of the Drawings

Reference is made, by way of example only, to the accompanying drawings in which:
FIGURE 1 is a flowchart of a general method of acquiring user state data in a wearable device;
FIGURE 2 is a flowchart of a detailed method of acquiring user data in a wearable device;
FIGURE 3 is an example time-series of primary raw sensor data, acquired from a 3-axis accelerometer;
FIGURE 4 is a flowchart of an example two-stage classification process;
FIGURE 5 is a hierarchical depiction of a user's resultant state and underlying sub-states;
FIGURE 6 is a first example emotional profile of a user, representative of the user's resultant state;
FIGURE 7 is a second example emotional profile of a user, representative of the user's resultant state;
FIGURE 8 is a flowchart of an example anomaly detection process;
FIGURE 9 is an example time-series of activity sub-state classification and environmental sub-state classification, as performed by a wearable device;
FIGURE 10 is a schematic diagram of electronic blocks underlying a wearable device;
FIGURE 11 is a schematic diagram of a communication subsystem unit, namely a Bluetooth Low Energy (BLE) module;
FIGURE 12a is a rendered view of an upper portion of an example wearable device;
FIGURE 12b is a rendered view of a lower portion of an example wearable device;
FIGURE 13 is a set of line-depictions of the exterior of an example wearable device; and
FIGURE 14 is a schematic diagram of a computing device capable of embodying hardware aspects of the invention.

### Detailed Description

The method and device described herein support the collection and processing of a plurality of clinical-grade biometric, biosignal, and kinesiological data, as well as environmental data, during extended periods. Devices may be suitable for home-use and in laboratory or clinical settings.

The use of on-board state classification (e.g., using artificial intelligence (Al)) enables autonomous decisions to be made within a small, self-powered wearable device. By using dynamic activity classification, techniques may achieve any or all of the following:
1) minimise the use of stored electrical energy to enable continuous collection of selected, relevant biosignals and environmental data;
2) reduce and compress collected signal data, store key extracted features, and spontaneously redact private activities;
3) optimise sensor activation and measurement frequency based on classification/prediction of users' activities;
4) suspend data collection during private/sensitive activities to preserve users' privacy; and
5) in combination, enable prolonged (for instance up to 6 weeks) unattended study, based on commercially available battery, data storage, and sensor technologies.

Commercial applications for this invention include health-, well-being-, mood-, fitness- and emotion-monitoring. In other aspects, personalised sports training, health monitoring, medical diagnostics, and clinical trial monitoring are possible.

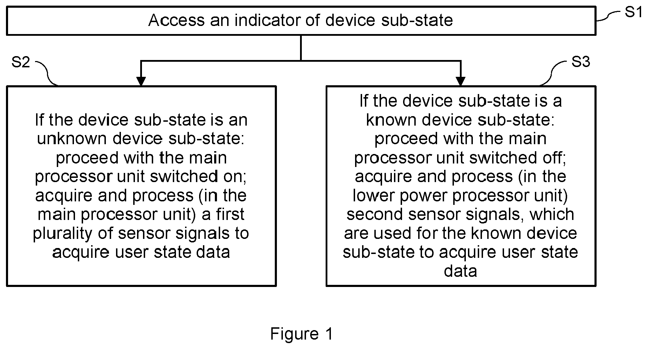

FIGURE 1 is a flow chart, detailing a method of acquiring user state data in a wearable device. The wearable device comprises at least a main processor unit and a lower-power processor unit.

At S1, the method accesses an indicator of device sub-state. If the sub-state is unknown, the method proceeds to S2, in which the main processor unit is switched on. The method then acquires and processes (in the main processor unit) a first plurality of sensor signals to acquire user state data.

If the device sub-state is a known device sub-state, the method proceeds to S3 with the main processor unit switched off. The method then acquires and processes (in the lower-power processor unit) a second plurality of pre-defined sensor signals, which are specific for the known device sub-state to acquire user state data.

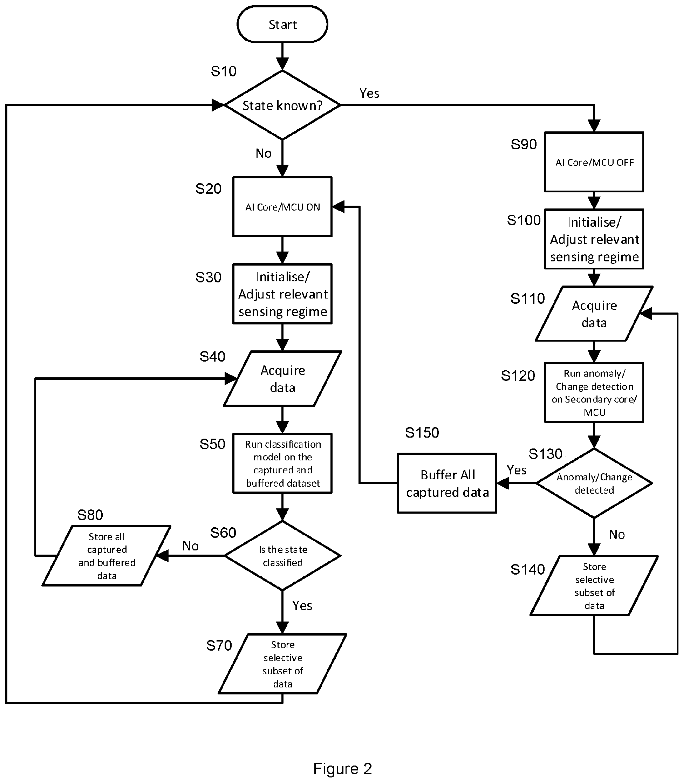

FIGURE 2 is a flowchart of an example method of data acquisition in a wearable device.

At S10, the wearable device assesses if the device sub-state is currently known. Specifically, the assessment is performed by a lower-power processor unit, which is the only processor unit active at this time. The device sub-state corresponds to a predefined mode of operation that sets the wearable device's operational parameters. These parameters include (but are not limited to):
a. The activation and deactivation states of sensors and communication units (e.g., BLE and Wi-Fi components) included within the wearable device;
b. Sampling rates of activated sensors;
c. Storage policies, defining the subset of captured data to be stored; and
d. Advertising schedules of the communication units.

These parameters are determined based on the importance and expected quality of each signal in the specific classified scenario that applies to the user during that state.

Where the device is turned on for the first time (where there is no information available to inform any state classification), the wearable may be configured to determine that the device sub-state is not currently known.

In the event that the wearable device determines that the device sub-state is not currently known, the processing continues via step S20. At S20, the wearable device powers on a higher-power processor unit. The main processor unit is initialised to start the process of state classification. In this example, the main processor unit is referred to as "AI Core/MCU" (microcontroller unit); the skilled reader will appreciate that, generally, the main processor unit need not be configured specifically for Al-processing and need not be a sole core of a processing unit.

At S30, the wearable device powers on all sensors (or at least a sufficient number of sensors to enable state classification). The wearable device initialises the sensors to run (e.g., acquire sensor data) at a predefined maximum output rate. Of course, the predefined output rate may vary for each sensor.

At S40, all sensors (including communication units) provide primary raw data, which the wearable device stores in an internal buffer. Where previously acquired data are present in the buffer, the wearable device appends newly acquired primary data to this previously acquired data.

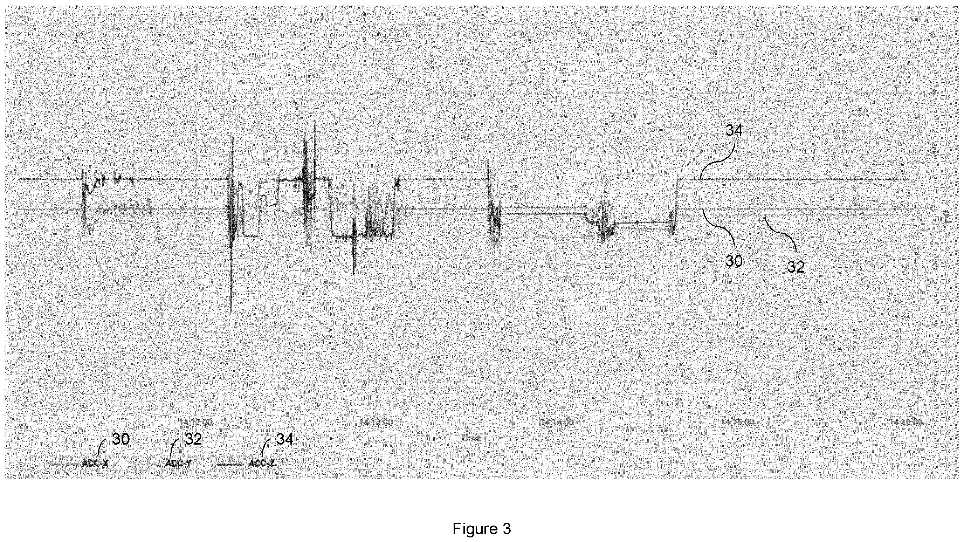

FIGURE 3 is example primary raw sensor data, acquired from a 3-axis accelerometer in the wearable device. The data comprises time-series for each orthogonal axis (namely, the x-axis acceleration 30, y-axis acceleration 32, and z-axis acceleration 34) of the accelerometer, measured in milli-g (mG). As seen in the plot, the user's acceleration in all three axes is initially constant before varying at intermittent intervals (notably at approximately time 14:12:10 onwards).

Returning to FIGURE 2, at S50, the wearable device (for example, the main processor unit thereof) runs a classification model on the acquired primary data to determine the device state and to provide an assessment of the user's resultant state. The wearable device carries out secondary signal calculations and feature extraction steps on the primary raw data, stored in the buffer. The wearable device stores these derived datasets alongside the primary raw data in the internal buffer to be used by a classifier.

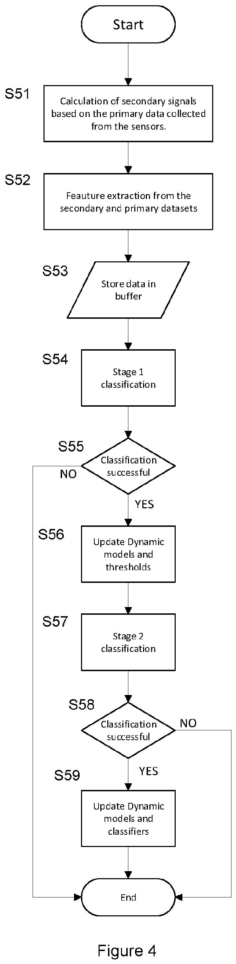

An example classification model may comprise two separate stages of classification. Initially, the wearable device may apply separate pretrained classification models to the data in the buffer to classify parameters such as user's activity, location, and environment. The wearable device may then carry out a second layer of classification using the outcome of the initial stage and using the data in the internal buffer as input parameters to determine the device state and to provide an assessment of the user's resultant state. FIGURE 4 is a detailed flowchart of an example two-stage classification process.

At S51, the wearable device processes the primary data collected from the sensors using the relevant processing techniques for each data stream to calculate the secondary values. For instance, heart rate, orientation, respiration rate, blood oxygen level, etc.

In addition, the wearable device may combine sensor streams using sensor fusion techniques to produce additional secondary outputs. For example, the wearable device may calculate orientation from an IMU via sensor fusion, and the wearable device may calculate R-R interval based on an ECG signal.

At S52, the wearable device extracts a subset of features from the primary and/or secondary streams to assist the classification. These features are typically created by applying mathematical or statistical operations on the dataset (for example, maximum, minimum, logarithm, etc.). It is not necessary for these to each have an established underlying physical meaning, as they are solely used by AI algorithms to classify the user's resultant state.

At S53, the wearable device stores the outputs from S51 and S52 (namely, primary and/or secondary data and subsets thereof) in a buffer to be processed by the classifiers.

At S54, the wearable device performs a first layer of classification. During this stage, pretrained classification models based on machine learning techniques, for instance, (e.g., CNN, Convolutional Neural Networks) are applied to the data stored in the buffer to determine the sub-state of the user's resultant state. The classification models may be pretrained using conventional AI frameworks, for instance Tensorflow or Keras. The wearable device stores results of this first layer of classification in a buffer.

As an example first layer of classification, the wearable device may implement human activity state classification as outlined by Jordao et al. in the 2018 work "Human Activity Recognition Based on Wearable Sensor Data: A Standardization of the State-of-the-Art".

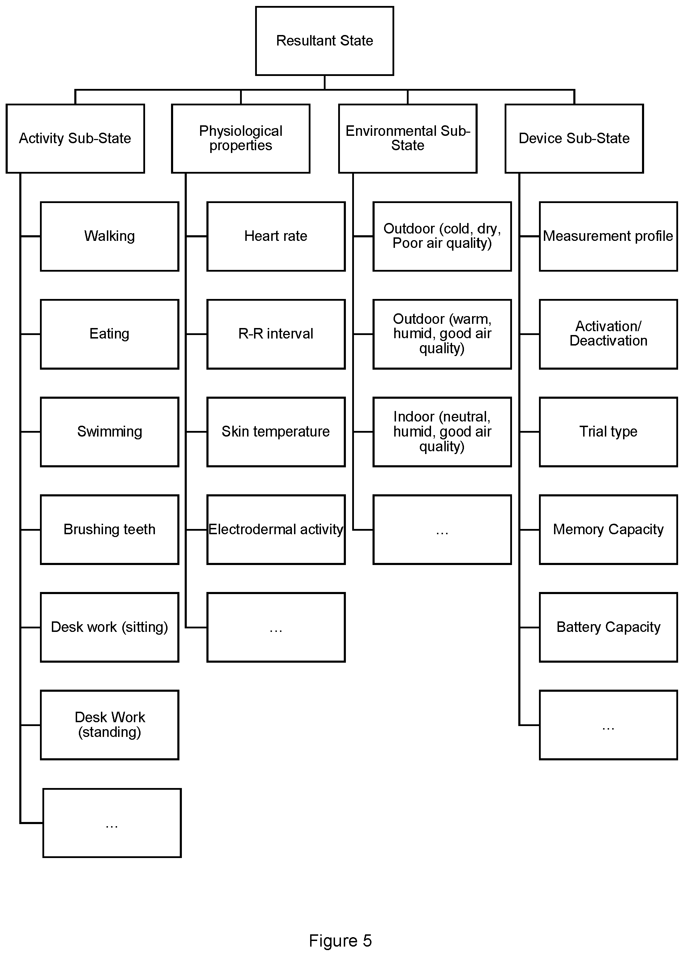

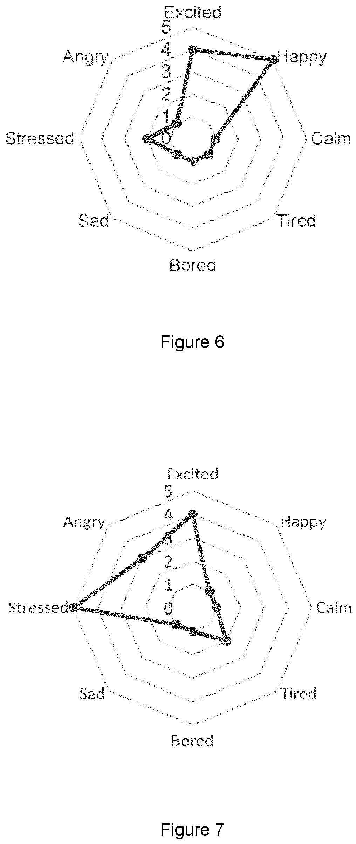

FIGURE 5 is a hierarchical overview of the user's resultant state, formed from an activity sub-state, physiological properties (or physiological sub-state), environmental sub-state, and the device sub-state. The wearable device calculates the resultant state (using AI algorithms) based on the classification of the sub-states and properties, including properties relating to the activity, well-being, emotional profile, and environment of the user. An example of an aspect (an emotional profile) of a user's resultant state is shown in FIGURE 6, where the user may be described as "overwhelmingly happy and excited, working at desk (sitting) in a pleasant indoor environment".

The activity sub-state is a classification of the activity that the user is currently carrying out. This is derived (using AI algorithms) based on the output of the sensors and secondary datasets (kinesiological, environmental, bio-signal, etc.). Example activity sub-states include walking, eating, swimming, brushing teeth, desk work (sitting), desk work (standing), showering, sleeping, gym (running), outdoor (running), cycling, driving (passenger), driving (driver), etc.

The physiological properties (or physiological sub-state) include a classification of the directly and indirectly (primary, secondary) sensed physiological parameters of the user. Examples include heart rate, R-R interval, skin temperature, electrodermal activity, etc.

The environmental sub-state is a classification of the current environment that the user is situated within, based on the environmental sensors. This may include both the type and the condition of the environment (as properties of the environment). Example environmental sub-state classifications include: outdoors, where the temperature is cold, humidity is low, and the level of airborne particulate matter and/or pollutants is high; outdoors, where the temperature is hot, humidity is high, and the level of airborne particulate matter and/or pollutants is low; indoors, where the temperature is average, humidity is high, and the level of airborne particulate matter and/or pollutants is low; etc.

The device sub-state is an indicator of the current state of the device, based on predefined settings and utilisation levels of the resources of the wearable device (e.g., battery and memory). This sub-state also includes the current, active measurement profile (e.g., which sensors are active and their data acquisition properties). This sub-state is usually set by the outcome of the resultant state in order to optimise the operation of the device (e.g., memory, power, and contextual relevance of the measurement profile). The device sub-state may also include information on the trial type, for instance continuous, event-activated, delayed start, sleep study, diurnal activity specific. The wearable device may be used for sports training, health monitoring, medical diagnostics, and/or clinical trial monitoring. The active trial type will guide the active or inactive on-board sensors.

Returning to FIGURE 4, at S55, the wearable device assesses the confidence level(s) of the output(s) of the first layer of classification. The wearable device may compare the confidence level(s) with predefined thresholds for each classification category (that is, for each user sub-state). If the classification of the sub-state is successful, the wearable device may move to the next stage of processing. If the classification is unsuccessful, the wearable device ends the classification procedure.

At S56, if necessary, the wearable device updates dynamic thresholds and models based on the results of the first layer of classification. This helps to ensure that the system is able to respond to individual characteristics of each user and "learn" and adapt to each scenario. For example, the wearable device may update the resting heart rate threshold for a specific user following classification of user activity sub-state "sleeping". The necessity of dynamic updating is dependent on the specific models used in the first layer of classification.

At S57, the wearable device applies a second layer of classification based on pretrained NN classifier. The outcome of the first layer of classification, primary data, secondary data, and extracted features stored in the buffer are input into the second layer of classification to determine the resultant user state.

As an example, the classification may determine the user activity sub-state to be "desk working", the environmental sub-state to be "hot/humid office", and the physiological sub-state to indicate elevated heart rate and higher stress indicators (based, for instance, on heart rate variability). The second layer of classification then may use all this information to classify the user's resultant state as "overwhelmingly stressed and excited, working at desk (sitting) in an unpleasant indoor environment". Of course, the resultant state may be stored as a tabular collection of marked and scored sub-states or in any other suitable format. This may be interpreted or displayed using text or graphical representation (for example on an external processing device). Example user graphical representations of the user's resultant state (in particular, the emotional profiles) are shown in FIGURE 6 and in FIGURE 7.

At S58, the wearable device assesses the confidence level(s) of the output(s) of the second layer of classification. The wearable device may compare the confidence level(s) with predefined thresholds for each classification category (that is, for each user sub-state). If the classification of the sub-state is successful, the wearable device may move to the next stage of processing. If the classification is unsuccessful, the wearable device ends the classification procedure.

At S59, if necessary, the wearable device updates dynamic thresholds and models based on the results of the second layer of classification. This process may occur as described above in respect of S56.

Returning to FIGURE 2, at S60, the wearable device assesses the outcome of the classification with a threshold confidence level. If the threshold is satisfied, the resultant state of the user is classified, and the wearable device moves on to the next stage of the process (S70).

When the classification is unsuccessful, at S80, the wearable device stores all the raw data in the memory for further, future investigation, and the clears the buffer. That is, in this scenario, the wearable device is uncertain as to the current resultant state of the user and therefore stores all data, which may be investigated at a later point in time.

When the classification is successful, at S70, the wearable device stores data in memory. With a successful classification of resultant state, only a subset of the data used for classification relevant to the resultant state is stored. For instance, where the user is classified as being - amongst other classifications - sedentary, there is no need for the wearable device to store, for example, ECG data sampled at a high frequency. Data that is deemed unnecessary to store is discarded; in this way, memory requirements of the wearable device are kept to a minimum.

To determine which portion of the data is to be stored following a successful classification, the wearable device is equipped with preconfigured profiles (an aspect of the device-sub state). For instance, for all classifications where the activity sub-state is classified as "desk work (sitting)", the associated preconfigured profile may indicate that the wearable device need not store high frequency ECG data.

If necessary, the wearable device may update classification thresholds and classification model parameters. In some scenarios, although the classification models may classify the sub-states confidently, one or more of the parameters may be outside of a predefined boundary or above or below threshold values. In these scenarios, these boundary and/or threshold values are updated to adapt to the new conditions and boundaries. This helps the wearable device to adapt to underlying physiological differences in user, how the users carry out certain activities, and/or how the users react to different events or stimuli. For example, an activity may be classified as running but the user is running excessively fast compared to predefined boundaries for acceleration. Therefore, in this case, the device may update acceleration boundaries to reflect this variance.

Preferably, the wearable device stores unclassified raw data with a label or labels, indicative of characteristics of the data signals. In this way, in the event that the wearable device fails to classify the resultant state of the user in future, but the underlying data characteristics are similar to those from a previously failed classification, the wearable device may quickly associate the similar data from multiple different failed classifications. Further, in this way, where the wearable device compiles numerous datasets from unclassifiable states and datasets may be reduced without loss of significant information, the wearable may perform such reduction. For example, if the wearable device acquires multiple unclassified datasets sharing similar data characteristics from all sensors, but the IMU sensor data indicates little or no movement from the user, the wearable may delete these data without impacting eventual classification of this state.

Following successful classification of the user's resultant state and storing of the selective subset of data in accordance with that subset, the wearable device returns to assess if the device sub-state is currently known at S10.

In the event that the wearable device determines that the device sub-state is currently known, the processing continues via step S90. At S90, the wearable device powers off the higher-power processor unit. Measurements and communication responsibilities are therefore delegated to the lower-power processor unit.

At S100, the wearable device activates and initialises (and deactivates, as necessary) the sensors and communication modules required for the current device sub-state. This is based on the predefined profile dictated by the device sub-state.

At S110, the wearable device acquires primary raw data from all activated sensors and communication devices. The wearable device stores these data in the internal buffer. If previous data are present in the buffer, the wearable appends the newly acquired data to the previous data.

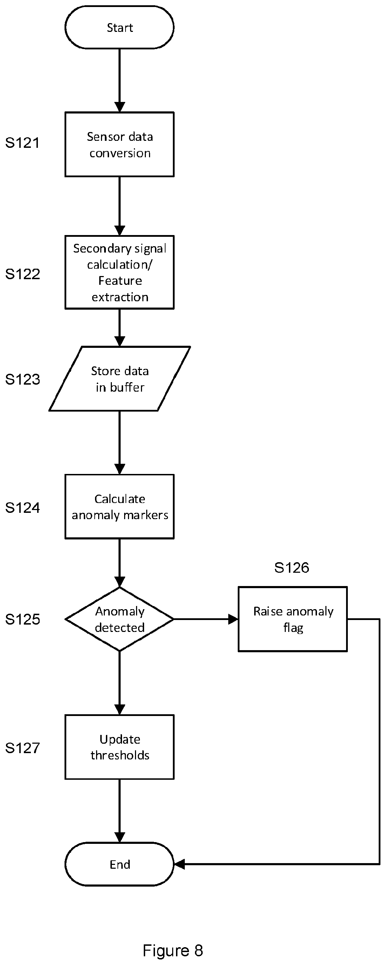

At S120, the wearable device performs secondary signal calculations and feature extraction steps upon the primary raw data. The wearable device stores these derived datasets alongside the primary raw data in the internal buffer. The wearable device applies an anomaly detection algorithm to data in the buffer to detect any divergence from expected values for the current state of the device, thereby potentially indicating a change of state. In turn, a change of state may necessitate a change in, e.g., active sensors in order to acquire sufficient data to characterise the resultant state of the user. FIGURE 8 is a detailed flowchart of an example anomaly detection process.

At S121, the secondary processor unit collects primary raw data. The wearable device converts this measurement data into correct, uniform values using standard techniques such as unit conversion and calibrations.

At S122, the secondary processor unit calculates secondary values based on the output of converted primary raw data. These values are calculated for each sensor (or combination of sensors) based on the nature of that specific sensor. For example, the secondary processor unit calculates orientation from the IMU via sensor fusion and calculates R-R interval based on the ECG signal.

At S123, the wearable device stores the outputs of S121 and S122 in the buffer to be processed by the anomaly detection algorithm.

At S124, the wearable device calculates anomaly markers using algorithms which require only low processing power, such as standard statistical methods. For example, the secondary processor unit calculates moving averages, windowed ranges, standard deviations, etc.

At S125, the wearable device compares the anomaly markers with predefined acceptable thresholds for the current state. These thresholds may be static (for example, the acceleration of a user exceeds a predefined absolute value) or may be adaptive (for example, the acceleration of a user exceeds a historical 90th percentile value). If necessary, additional measurements are taken to confirm the anomaly. For instance, depending on the confidence level of the algorithm, extra measurements from additional sensors (which were not used initially in the state) might be needed. As an example, where the orientation of the user is not accurately determined using a 3-axis accelerometer, the wearable device may initiate a 6-axis or even 9-axis IMU solely to resolve this uncertainty. As another example, depending on the confidence level of the algorithm, measurement from active sensors may be taken as an increased rate of measurement.

In the event that the anomaly algorithm determines that the acquired data are anomalous (in respect of the current user device sub-state), at S126, the wearable device raises a flag to indicate that an anomaly is detected.

In the event that the anomaly algorithm determines that the acquired data are not anomalous (e.g., anomaly markers are within an acceptable threshold range), at S127, if necessary, the wearable device updates any dynamic or adaptive threshold. The wearable device then moves on to the next stage of processing.

Returning to FIGURE 2, at S130 the wearable device compares the confidence level of the output from the anomaly detection algorithm with a threshold to determine if an anomaly is detected.

In the event that no anomaly is detected, at S140, the wearable device stores a subset of the raw data relevant to the current device sub-state in the memory and the buffer is cleared. In addition, relevant adaptive parameters of the anomaly detection algorithm are updated with the new data (if necessary). The wearable device then returns to S110, where the wearable device continues to acquire primary raw data from all activated sensors and communication devices.

In the event that an anomaly is detected (as indicated, for instance, using the flag from S126), at S150, the wearable device stores all captured data in the buffer for further investigation. The wearable device then proceeds to S20 and S30 and so on, where the wearable device powers on a higher-power processor unit and all sensors. In this way, the buffered anomalous data may be further investigated, using the higher-power processor unit.

To elucidate the anomaly detection mechanism, consider the two following example scenarios. In the first example scenario, the current device sub-state is known and indicates a sleep measurement profile, and the user activity sub-state is known to be "sleeping". Note that the other sub-state information, such as environmental sub-state, is not relevant for the purpose of this example scenario. During measurement cycles, markers calculated based on a low power accelerometer indicate an increase in kinesiological activity of the user, which is outside of the acceptable threshold of the current state. This acceptable threshold is predefined based on prior measurements. One example of these markers is the windowed moving average of resultant linear acceleration amplitude. As a result, the wearable device raises a flag indicating an anomaly. This potentially indicates that the user is waking up; control is passed to primary processor unit and sensors of the wearable to investigate this further.

In the second example scenario, the current device state is known and indicates a general measurement profile, the activity sub-state is known to be "desk work (sitting)", and the emotional profile (determined from resultant state of the user) is known to be generally neutral. Again, note that the other sub-state information, such as environmental sub-state, is not relevant for the purpose of this example scenario. During measurement cycles, primary and secondary bio-signals indicate a change in the monitored markers outside of the thresholds while other markers such as kinesiological signals and environmental parameters are not indicating an anomaly. Examples of these bio-signal markers may be a short span HRV (Heart Rate Variability), change in heart rate, change in Pulse Transient Time (PTT), change in GSR, and change in skin temperature. These values may be compared with static baseline threshold for the specified state or adaptive threshold (e.g., skin temperature). These anomalies may therefore indicate a change in emotional state of the user and therefore a flag will be raised. In this way, the main processor unit - with greater processing power than the secondary processor unit - may investigate and classify the new state.

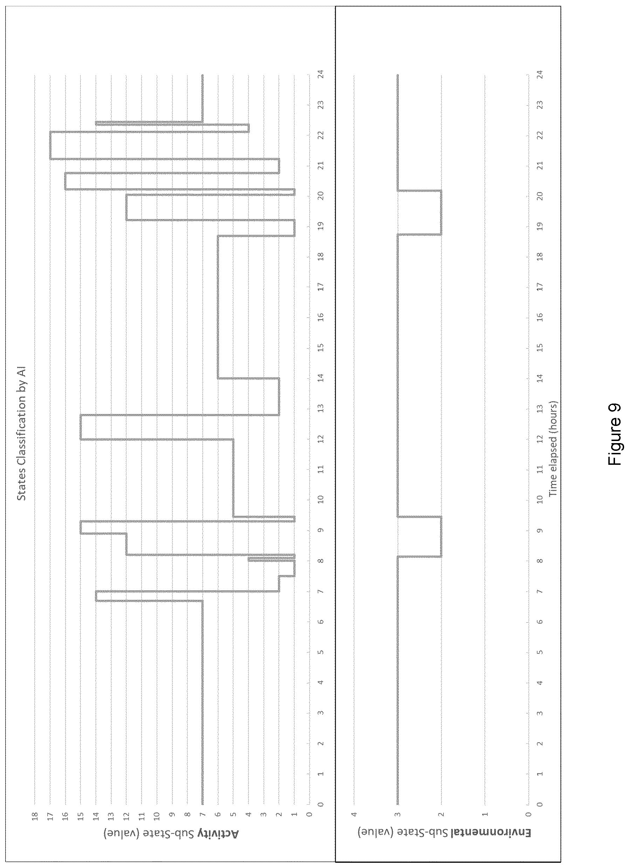

FIGURE 9 is a time-series of state classifications as performed by a wearable device. The upper panel illustrates the transition between activity sub-states over a 24-hour period. The lower panel illustrates the transition between environmental sub-states over the same 24-hour period. For instance, at launch (at time zero), the wearable device classifies the wearer as being in activity sub-state denoted by index 7 and in environmental sub-state denoted by index 3. TABLE 1 below provides a summary of the relevant activity sub-states and environmental sub-states in a portion of this example.

**TABLE 1, dynamic sub-state classification**

| Time elapsed (h) | **Activity Sub-state** | | **Environmental Sub-state** | |
|---|---|---|---|---|
| | Value | Description | Value | Description |
| 0.00 | 7 | Sleeping | 3 | Indoor (neutral, humid, good air) |
| 6.70 | 7 | Sleeping | 3 | Indoor (neutral, humid, good air) |
| 6.70 | 14 | New (unknown) | 3 | Indoor (neutral, humid, good air) |
| 7.00 | 14 | New (unknown) | 3 | Indoor (neutral, humid, good air) |
| 7.00 | 2 | Eating | 3 | Indoor (neutral, humid, good air) |
| 7.50 | 2 | Eating | 3 | Indoor (neutral, humid, good air) |
| 7.50 | 1 | Walking | 3 | Indoor (neutral, humid, good air) |
| 8.00 | 1 | Walking | 3 | Indoor (neutral, humid, good air) |
| 8.00 | 4 | Brushing Teeth | 3 | Indoor (neutral, humid, good air) |
| 8.10 | 4 | Brushing Teeth | 3 | Indoor (neutral, humid, good air) |
| 8.10 | 1 | Walking | 3 | Indoor (neutral, humid, good air) |
| 8.15 | 1 | Walking | 3 | Indoor (neutral, humid, good air) |
| 8.15 | 1 | Walking | 2 | Outdoor (warm, humid, good air) |
| 8.20 | 1 | Walking | 2 | Outdoor (warm, humid, good air) |
| 8.20 | 12 | Driving (passenger) | 2 | Outdoor (warm, humid, good air) |
| 8.90 | 12 | Driving (passenger) | 2 | Outdoor (warm, humid, good air) |
| 8.90 | 15 | New (unknown) | 2 | Outdoor (warm, humid, good air) |
| 9.30 | 15 | New (unknown) | 2 | Outdoor (warm, humid, good air) |
| 9.30 | 1 | Walking | 2 | Outdoor (warm, humid, good air) |
| 9.45 | 1 | Walking | 2 | Outdoor (warm, humid, good air) |
| 9.45 | 5 | Desk work (sitting) | 3 | Indoor (neutral, humid, good air) |
| 12.00 | 5 | Desk work (sitting) | 3 | Indoor (neutral, humid, good air) |
| 12.00 | 15 | (unknown) | 3 | Indoor (neutral, humid, good air) |
| 12.80 | 15 | (unknown) | 3 | Indoor (neutral, humid, good air) |
| 12.80 | 2 | Eating | 3 | Indoor (neutral, humid, good air) |
| ... | ... | ... | | ... |

That is, just before 7 hours following launch of the wearable device, the user activity sub-state is reclassified from "sleeping" (index 7) to an unknown state (index 14). The environmental sub-state remains unchanged at this time ("indoor (neutral, humid, good air)"; index 3). As described above, as the user activity sub-state is unknown at this time, the wearable device makes the decision to power on the higher-power processor unit and acquires data from all sensors, allowing for classification of this activity sub-state at a later time.

At 8.15 hours following launch of the wearable device, the environmental sub-state is reclassified from "indoor (neutral, humid, good air" (index 3) to "outdoor (warm, humid, good air)".

After 8.90 hours following launch of the wearable device, the user activity sub-state is reclassified to a second unknown state (index 15). At 12.00 hours following launch of the wearable device, the user activity sub-state is again reclassified to this second unknown state. As described above, the wearable device is determining in this case that the data characteristics are similar to those from a previously failed classification.

The skilled reader will appreciate that the particular described sub-states are examples and further and/or more granular sub-states are realisable. Further, the physiological sub-state and the device sub-state (or aspects thereof) may be similarly plotted/tabulated. For example, an indication of which sensors are active for a particular activity sub-state (an aspect of the device sub-state) may be similarly plotted/tabulated.

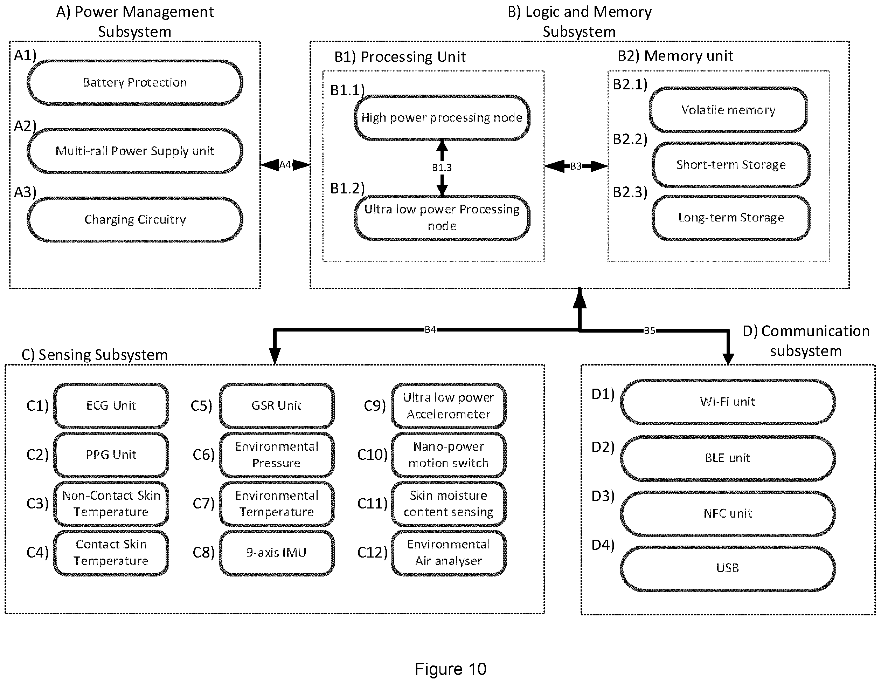

FIGURE 10 is a schematic diagram of functional electronic blocks underlying an example wearable device, used in the processing outlined above. Broadly, the electronic components may be categorised into four main subsystems: power management; logic and memory; sensing; and communication. The skilled reader will appreciate that additional functional subsystems and additional units (or, alternatively, a subset of the described example subsystems and units) may be incorporated into wearable devices with no loss of generality.

The power management subsystem A has three separate units, which are responsible for battery protection, charging and regulation.

The battery protection unit A1 is responsible for protecting the battery against short circuits, over current, and over/under voltage.

The multi-rail power supply unit A2 is responsible for providing the necessary power to the higher-power processor unit and other subsystems. It does this by converting the voltage supplied by the battery into suitable voltage rails (levels) for each component. In addition, this subsystem has the capability to disable a specific rail in order to save power if it is required by the processor.

The charging circuitry unit A3 is responsible for safely charging the device battery. Any known device battery technology may be used (e.g., alkaline, Li-ion, or LiPo batteries). Rechargeable battery technologies (e.g., Li-ion) are advantageous in that the device need not be disposed following battery depletion. The power management subsystem A may be supplemented by means to recharge rechargeable batteries, such as motion-powered charging means or solar charging means.

The logic and memory subsystem B is connected to the power management subsystem A via connection (bus or interface) A4 in order to control voltage rails and sense battery level, charging status, and current battery consumption.

The logic and memory subsystem B is responsible for running the embedded code/algorithms, acquiring data from sensors, and storing the collected data in memory. This subsystem may be functionally divided into two main units of a processor unit B1 and a memory unit B2.

The processor unit B1 comprises at least two separate processor units; namely a main or higher-power processor unit and a secondary or lower-power processor unit. These processor units may either be on separate chips or part of a multi core chip. Regardless of their configuration, they may be separated into two separate types of high power and low power processor units.

High power processor unit (main or primary core; main controller) B1.1 has a significantly higher processing capability compared to the low power processor unit B1.2. The higher-power processor unit also consumes more power compared to the lower-power processor unit. The high processing capabilities of this processor unit make it suitable for running the complex embedded AI classification algorithms or tasks, which typically have high a processing load (e.g., requiring fast data transfer). However, the higher power consumption of this processor unit makes running them continuously impractical.

The lower-power processor unit (lower-power secondary core; lower-power controller) B1.2 has a significantly lower power consumption compared to the main processor unit B1.1. This makes the lower-power processor unit B1.2 suitable for tasks that have lower processing load (e.g., sensor data acquisition or anomaly detection) or need to be running continuously.

Both processor units are connected to each other via interrupt routines and a dedicated data bus B1.3. This enables handoff of operations to take place between the processor units during operational state transitions.

The processor unit B1 mainly uses the memory unit B2 to store the raw and processed data (e.g., collected from the sensors) and operational data (e.g., errors and logs). In addition, the processor unit B1 (lower-power processor unit B1.2) uses the memory unit B2 to temporarily store data from the sensors (i.e., buffer) for further processing or classification by the main processor unit B1.1.

The volatile memory unit B2.1 of the memory unit B2 is used for temporary storage of data by the processor units during calculations.

The short-term storage unit B2.2 is used for short term storage of datasets (larger than the capacity of the volatile memory). This is mainly used at the initial stage of data collection before data are binned/classified or during handover of data from the lower-power processor unit B1.2 to the higher-power processor unit B1.1.

The long-term storage unit B2.3 is the main storage unit of the device, which is used for storage of data and logs for further processing and analysis (by external processing device, e.g., PC).

A high-speed data bus B3 connects the processor unit B1 and the memory unit B2.

A logic/memory-sensing data bus, Interrupt, and control channel B4 connects logic and memory subsystem B and the sensing subsystem C. This is used to collect measurements (for storage and for processing) and to configure and control the sensors.

A logic/memory-communication data bus and control channel B5 connects the logic and memory subsystem B and the communication subsystem D. This is used during data transfer (e.g., Device-Device data transfer or Device-PC data transfer).

The sensing subsystem C comprises a variety of sensors that are used to collect specific operational, environmental, bio-signal and activity parameters.

An ECG (electrocardiogram) sensor unit C1 is responsible for measurement of cardiac signals via ECG contact electrodes.

A PPG (photoplethysmography) sensor unit C2 is responsible for measurement of cardiac pulse via optical methods and may be in-sync with the ECG sensor unit C1.

A non-contact infrared (IR) based temperature sensor unit C3 is used to measure skin surface temperature.

A contact-based temperature sensor unit C4 is used to measure skin/body temperature.

A GSR (Galvanic Skin Response) sensor unit C5 is used to monitor changes in the activity of sweat glands via measuring changes in skin electrical conductivity.

Environmental atmospheric pressure sensor unit C6 and environmental temperature sensor unit C7 are used to monitor environmental properties (pressure and temperature, respectively) in the proximity of the user.

A 9-axis IMU unit C8 comprising a high performance 3-axis accelerometer, 3-axis magnetometer, and 3-axis gyroscope is used to measure detailed kinesiological parameters. In addition, this unit may include an embedded low power processor unit in order to carry out sensor fusion algorithms (e.g., orientation calculations) and light activity classification (e.g., running, walking, standing) separately from the main logic subsystem B1. In this way, the power consumption of the wearable device (e.g., incurred through otherwise unnecessary activation of the higher-power processor unit B1.1) may be reduced.

A 3-axis accelerometer unit C9 is used to provide less detailed kinesiological information (in comparison to that provided by the 9-axis IMU C8) during operational states in which the IMU data are not required. Of course, the skilled reader will appreciate that the wearable device may additionally or alternatively include accelerometers configured to sense acceleration data with fewer or more axes (for instance, a 2-axis accelerometer and/or a 6-axis accelerometer).

A motion switch unit C10 is an ultra-low power (nano watt range) motion detection sensing system, which is used to detect motion when other motion detection systems (IMU C8, accelerometer C9) are not operational (and their activation and associated energy consumption would be surplus to requirement).

A skin moisture content sensing unit C11 is a sensing system used to measure relative skin moisture content.

An environmental air analyser unit C12 is used to detect and measure the presence and concentration of specific gasses, chemicals, or particles in the air (e.g., CO₂, NOₓ, alcohols, etc.).

The communication subsystem D is responsible for data transfer between devices and to/from the controlling device (e.g., PC, tablet). For instance, the wearable device may transmit stored data to external resources in order to investigate the stored data (e.g., to refine the on-board pretrained classification models, or to train new classification models based on acquired anomalous data). Further, the wearable device may acquire sensor signals from sensor units that are not part of the on-board sensing subsystem C, but are, instead, equipped with loT functionality.

A Wi-Fi unit D1 provides standard Wi-Fi connectivity to the device, which is used for fast data transmission and device setup.

A BLE (Bluetooth Low Energy) unit D2 provides low power communication between devices and PC/tablet for data transfer and setup.

An NFC (Near Field Communication) unit D3 is used for device pairing, device identification, and data transfer authorisation.

USB (Universal Serial Bus) D4 capability is used for large data transfer and device setup.

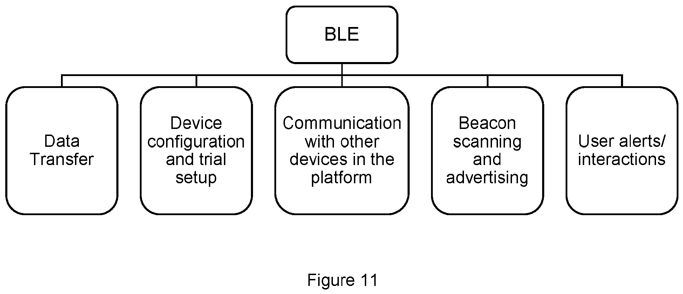

FIGURE 11 is a schematic diagram of the functionality of an example BLE unit D2. As described above, the wearable device may be equipped with a BLE module/capability. The BLE unit has an embedded lower-power processor unit that handles all of the overheads associated with the BLE protocol. This enables the BLE unit to operate independently of the main processor unit(s) when the BLE unit is required (e.g., in scenarios were both main and secondary processor units are in sleep/low power mode).

Data transfer is one of the main functions of the BLE unit. The stored data may be transferred to BLE enabled devices such as PCs, smartphones, or tablets for further investigation and for further processing.

The BLE unit may also be used to configure the wearable device. For instance, the BLE unit may be used during sensor calibrations, to accommodate firmware upgrades etc. Where the wearable device is to be used to acquire user state data for, e.g., a medical trial or an investigative trial, the BLE unit may be used to set up trial parameters such as the start time of the trial, the duration of the trial, and active and inactive sensors.

Where the wearable device is configured to acquire sensor signals from external devices, such external devices in the platform may also be equipped with a similar BLE unit. This enables communication between devices within the platform. Examples of such communication involve downloading of the external devices' data into the wearable device for future transmission from the wearable device to another external device for investigative processing, or for use by the on-board classification algorithm(s) of the wearable device.

For instance, BLE interaction with external devices may be used for synchronising and correlating device exposure with user behaviour and user reactions. As an example, the wearable device used in combination with an external treadmill equipped with BLE communication capabilities may enable linking and correlating usage of such gym equipment with the user's emotional state before and after the training session.

With certain device sub-states or when it is necessary to determine the state of the wearable device (e.g., when powering-on the device for the first time), the BLE unit may scan the RF environment for other Bluetooth devices that are nearby. This enables the wearable device to discover other devices used in the platform (active BLE usage beacons) and connect to them.

In addition, on-board pretrained machine learning classification models may use the type of the discovered external devices (third party/platform) and a proximity estimation of the external device (e.g., based on signal strength) to assist in classifying states. A simplified example of this involves discovery of an active BLE-enabled piece of gym equipment; when the wearable device senses higher than usual user activity levels and senses other fitness devices nearby, the confidence of a classification that indicates the user is exercising may be improved.

In addition to scanning for BLE external devices (beacons), the wearable device may advertise its own beacon for other platform devices. In this way, the wearable device itself may form part of a wider platform and may inform classifications in other wearable devices. That is, when a first wearable device (worn by a first user) advertises its presence to a second wearable device (worn by a second user), the second wearable device's classification may take into account that the first user is nearby; thus, the second wearable device may determine that the second user is likely in a social environment. Optionally, this functionality may be present only for/in certain device sub-states. For example, the wearable device may advertise its own beacon in all device sub-states in which the device does not need to conserve power; the beacon is stopped in non-active device sub-states, such as when the wearable device is confident that the user is sleeping or otherwise dormant.

The BLE unit may additionally or alternatively be used to send notifications or to communicate with users' BLE devices (e.g., a mobile phone) in order to provide useful information regarding the user's activity/state, and to capture user feedback or interactions with the user's device.

While the above discusses the BLE communication capabilities of the wearable device, the skilled reader will appreciate that this functionality may be provided with other means or protocols of communication. For example, acquisition of sensor signals from external devices may be performed using any suitable communication protocol, such as ZigBee, Li-Fi, NFC, RFID, LTE, 5G, etc.

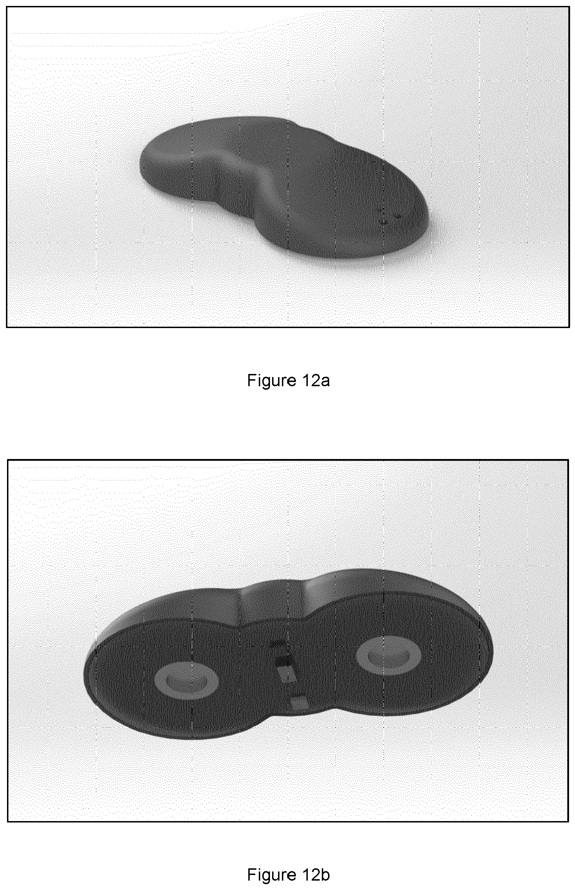

FIGURE 12a and FIGURE 12b are a pair of three-dimensional renderings of the exterior of a wearable device. The former rendering illustrates the low-lying profile of the device, suitable for a "fit-and-forget" approach, where the user may simply affix the device to the body (not shown) where it may remain until, for instance, sufficient data are acquired, or on-board battery supplies are exhausted. The latter rendering illustrates the skin-facing aspects of the wearable, including a pair of ECG electrode snap points and skin-facing sensors.

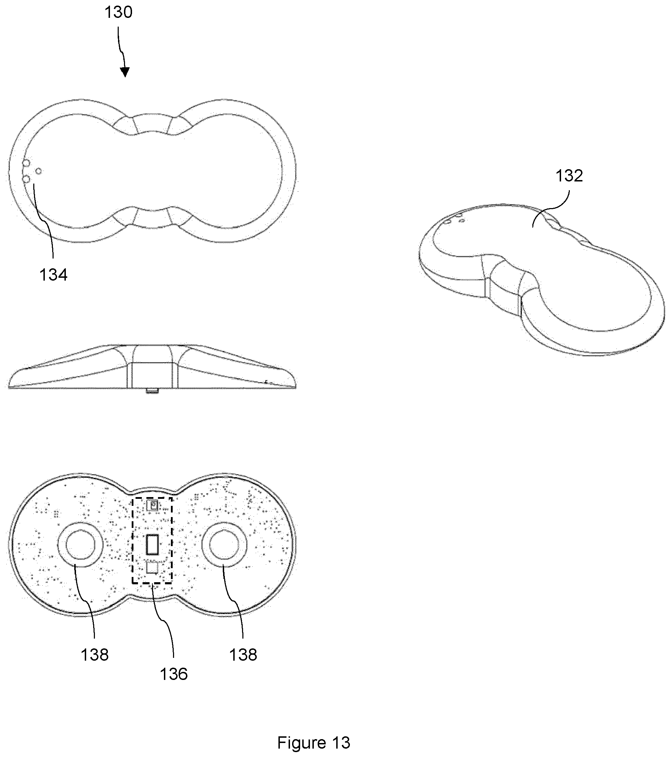

FIGURE 13 is a set of rendered line-depictions of the exterior of an example wearable device 130 (the same wearable as depicted in FIGURES 10a and 10b). The exterior shell 132 is a plastic shell designed to house the electronics and to protect them from damage while being comfortable to wear by the user. The vents 134 provide small holes in the shell 132 to enable use of environmental sensors (housed within the device, in a suitably sealer chamber). Skin facing sensors 136 comprise both contact and non-contact bio-signal sensors (sensors for e.g., skin temperature, PPG, IR based skin temperature, skin moisture content). ECG electrode snap points 138 are gold plated ECG electrode snap connectors, compatible with standard ECG electrodes.

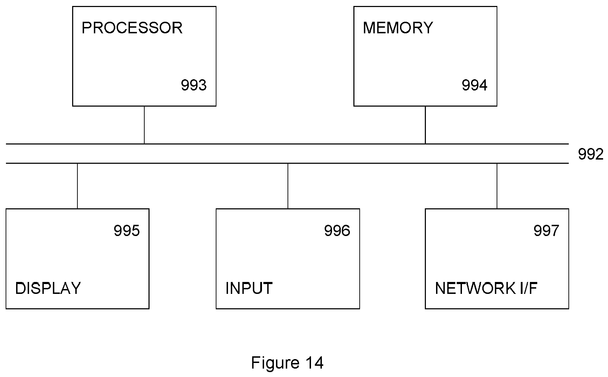

FIGURE 14 is a block diagram of a computing device, such as the computing aspects of a wearable device, which embodies the present invention, and which may be used to implement a method of an embodiment of acquiring user state data in the wearable device. The computing device comprises a processor 993, and memory 994. Optionally, the computing device also includes a network interface 997 for communication with other computing devices, for example with other computing devices of invention embodiments.

For example, an embodiment may be composed of a network of such computing devices. Optionally, the computing device also includes one or more input mechanisms such as GUI 996, and a display unit such as one or more screens or monitors 995. The components are connectable to one another via a bus 992.

The memory 994 may include a computer-readable medium, which term may refer to a single medium or multiple media (e.g., a centralized or distributed database and/or associated caches and servers) configured to carry computer-executable instructions or have data structures stored thereon. Computer-executable instructions may include, for example, instructions and data accessible by and causing a general purpose computer, special purpose computer, or special purpose processing device (e.g., one or more processors) to perform one or more functions or operations. Thus, the term "computer-readable storage medium" may also include any medium that is capable of storing, encoding, or carrying a set of instructions for execution by the machine and that cause the machine to perform any one or more of the methods of the present disclosure. The term "computer-readable storage medium" may accordingly be taken to include, but not be limited to, solid-state memories, optical media, and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

The processor 993 is configured to control the computing device and execute processing operations, for example executing code stored in the memory to implement the various different functions of subsystems and units (in particular, the main processor unit and the lower-power processor unit) described here and in the claims. The memory 994 stores data being read and written by the processor 993. As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more embodiments, a processor is configured to execute instructions for performing the operations and steps discussed herein.

The display unit 997 may display a representation of data stored by the computing device and may also display screens enabling interaction between a user and the programs and data stored on the computing device. The input mechanisms 996 may enable a user to input data and instructions to the computing device.

The network interface (network I/F) 997 may be connected to a network, such as the Internet, and is connectable to other such computing devices via the network. The network I/F 997 may control data input/output from/to other external devices and/or external processing devices via the network. Other peripheral devices such as microphone, speakers, visual alarm means, etc. may be included in the computing device.

The main processor unit (the higher-power processor unit B1.1 of FIGURE 10) may be a processor 993 (or plurality thereof) executing processing instructions (a program) stored on a memory 994 and exchanging data via a network I/F 997 or via input mechanism 996. In particular, the processor 993 executes processing instructions to receive, via the network I/F, configuration data from external devices and/or external processing devices, to configure the wearable device to capture user state data, and to execute user state classification, as in S20 to S80 of FIGURE 2. Furthermore, the processor 993 may execute processing instructions to store user state data on a connected storage unit and/or to transmit, via the network I/F 997, user state data to external processing devices for further investigation, for refinement of classification algorithms, or for development of new classification algorithms.

The lower-power processor unit (B1.2 of FIGURE 10) may be a processor 993 (or plurality thereof) executing processing instructions (a program) stored on a memory 994 and exchanging data via a network I/F 997 or via input mechanism 996. In particular, the processor 993 executes processing instructions to receive, via the network I/F, configuration data from external devices and/or external processing devices, to configure the wearable device to capture user state data, and to acquire user state data following successful state classification, as in S90 to S150 of FIGURE 2. Furthermore, the processor 993 may execute processing instructions to store user state data on a connected storage unit and/or to transmit, via the network I/F 997, user state data to external processing devices for further investigation, for refinement of classification algorithms, or for development of new classification algorithms.

Methods embodying the present invention may be carried out on a computing device such as that illustrated in FIGURE 14. Such a computing device need not have every component illustrated in FIGURE 14 and may be composed of a subset of those components. A method embodying the present invention may be carried out by a single computing device in communication with one or more data storage servers via a network. The computing device may be a data storage itself storing user state data.

A method embodying the present invention may be carried out by a plurality of computing devices operating in cooperation with one another. One or more of the plurality of computing devices may be a data storage server storing at least a portion of user state data.

## Claims

1. A computer-implemented method of acquiring user state data in a wearable device, the wearable device comprising a main processor unit and a lower-power processor unit, the method comprising:
accessing an indicator of device sub-state;
if the device sub-state is an unknown device sub-state, proceeding with the main processor unit switched on, and acquiring and processing in the main processor unit a first plurality of sensor signals to acquire user state data; and
if the device sub-state is a known device sub-state, proceeding with the main processor unit switched off, and acquiring and processing in the lower-power processor unit second sensor signals which are used for the known device sub-state to acquire user state data.

2. The method according to claim 1, wherein the second sensor signals comprise fewer sensor signals than the first plurality of sensor signals.

3. The method according to claim 1 or claim 2, wherein the main processor unit processes the first plurality of sensor signals by using at least one trained machine learning model to output a classification of the user state and/or a resultant state of the user.

4. The method according to claim 3, wherein the main processor unit processes the first plurality of sensor signals by:
passing the first plurality of sensor signals through an initial trained machine learning model to output a classification of the user state according to pre-determined sub-states; passing the classification of the user state and the first plurality of sensor signals through a subsequent trained machine learning model to output a resultant state of the user.

5. The method according to claim 4, further comprising:
determining the confidence of the resultant state of the user, and
when the confidence is below a threshold, passing the first plurality of sensor signals to a memory of the wearable device for further investigation and continuing to acquire the first plurality of sensor signals.

6. The method according to any of claims 3 to 5, wherein the main processor unit updates adaptive parameters of the at least one trained machine learning model following output of the classification of the user state and/or the resultant state of the user.

7. The method according to any preceding claim, wherein the lower-power processor unit:
detects whether there is an anomaly when processing the second sensor signals;
initialises the main processor unit following detection of an anomaly; and
then passes the second sensor signals for processing in the main processor unit, optionally wherein processing in the main processor unit further acquires and uses the first plurality of sensor signals.

8. The method according to claim 7, wherein detection of the anomaly comprises:
passing the second sensor signals through an anomaly detection algorithm to detect divergence from expected values for the current device sub-state; and
detecting the anomaly when a confidence level of the anomaly detection algorithm output exceeds a predefined threshold.

9. The method according to claim 7 or claim 8, wherein the lower-power processor unit updates adaptive parameters of the anomaly detection algorithm following detection of the anomaly.

10. The method according to any preceding claim, further comprising:
following acquiring user state data, discarding at least a portion of the user state data, the portion of the user state data being irrelevant for the known device sub-state, wherein relevance of the user data to the known device sub-state is preconfigured for each device sub-state.

11. The method according to any preceding claim, wherein the main processor unit is switched on when the indicator of device sub-state is accessed, and the method further comprises:
if the device sub-state is unknown, leaving the main processor unit switched on; and
if the device sub-state is known, switching off the main processor unit.

12. The method according to any preceding claim, wherein the first plurality of sensor signals and/or the second sensor signals are acquired at least in part from at least one of the following: biometric sensors for sensing biometric signals of the user; environmental sensors for sensing local environmental properties; communication sensors; and kinematic sensors.

13. The method according to any preceding claim, wherein the user state data is a resultant state of the user determined according to at least one and preferably all of the following: activity sub-state of the user; biometric sub-state of the user; environmental sub-state; and the device sub-state.

14. The method according to claim 13, wherein the resultant state of the user comprises an emotional profile of the user.

15. The method according to any preceding claim, further comprising transmitting, by a communication subsystem, user state data to an external processing device.

16. The method according to any preceding claim, wherein the first plurality of sensor signals and/or the second sensor signals are acquired at least in part from an external device.

17. A wearable device having a main processor unit, a lower-power processor unit, and a memory, the main processor unit and the lower-power processor unit configured to execute the steps of the method of any preceding claim.

18. A computer program comprising instructions to cause the wearable device of claim 17 to execute the steps of the method of any of claims 1 to 16.

19. A computer-readable medium having stored thereon the computer program of claim 18.
